# EUROPEAN PATENT APPLICATION

(11) **EP 4 560 029 A1**
(43) Date of publication of application: **28.05.2025**
(21) Application number: 23383195.7
(22) Date of filing: 22.11.2023
(51) Int. Cl.: C12Q 1/6883

(54) **METHOD FOR THE DIAGNOSIS OF ANEURYSM, AND INHIBITORS FOR USE IN THE PREVENTION OR TREATMENT OF ANEURYSM**

(71) Applicant: Fundacion Instituto De Investigacion Sanitaria Fundacion Jimenez Diaz, 28040 Madrid (ES); Consejo Superior De Investigaciones Científicas - CSIC, 28006 Madrid (ES)
(72) Inventor: OLLER PEDROSA, Jorge, 28040 Madrid (ES); MITTELBRUM HERRERO, María, 28006 Madrid (ES)
(74) Representative: Hoffmann Eitle

(57) **Abstract**

The present invention refers to an *in vitro* method for the diagnosis of aneurysm which comprises assessing the level of a nucleotide sugar of the hexosamine biosynthetic pathway selected from the list comprising: Glucosamine-6-P, N-acetyl-glucosamine-6P, N-acetyl-glucosamine-1-P or UDP-N-acetylglucosamine, Galactosamine-6-P, N-acetyl-galactosamine-6P, N-acetyl-galactosamine-1-P or UDP-N-acetyl-galactosamine or of proteins that have been co-translationally or post-translationally modified trough the addition of the above nucleotide sugars. It also refers to an inhibitor of an enzyme involved in the hexosamine biosynthetic pathway selected from the group comprising: GFPT1, GFPT2, GNPNAT1, PGM3 and/or UAP1, and/or of an enzyme downstream the hexosamine pathway selected from: GALE, OGT, OGA, MGAT1, MGAT2, MGAT3, MGAT5B, B3GNT3, GNE, HAS1, HAS3 and/or enzymes involved in the integrated stress response (ISR), for use in the prevention and/or treatment of aneurysm.

## Description

### FIELD OF THE INVENTION

The present invention refers to the medical field. Particularly, the present invention refers to an *in vitro* method for the diagnosis of aneurysm which comprises assessing the level of a nucleotide sugar of the hexosamine biosynthetic pathway selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine or of proteins that have been co-translationally or post-translationally modified trough the addition of the above nucleotide sugars. It also refers to an inhibitor of an enzyme involved in the hexosamine biosynthetic pathway selected from the group comprising: GFPT1, GFPT2, GNPNAT1, PGM3 and/or UAP1, and/or of an enzyme downstream the hexosamine pathway selected from: GALE, OGT, OGA, MGAT1, MGAT2, MGAT3, MGAT5B, B3GNT3, GNE, HAS1, HAS3 and/or enzymes involved in the integrated stress response (ISR), for use in the prevention and/or treatment of aneurysm.

### STATE OF THE ART

The aorta is the body's largest artery. It carries oxygen-rich blood from the heart to the rest of the body. This blood vessel originates in the heart's left ventricle, or upper chamber, and then curves upward into the chest before bending downward into the abdomen, where it splits into two arteries that carry blood to each leg. Other arteries branch off from parts of the aorta to carry blood to the upper body and to organs such as the kidneys, stomach, and intestines.

The wall of the aorta is composed of three layers. If the middle layer of the aortic wall - which consists of smooth muscle and elastic tissue - weakens and stretches, blood pumping through it can lead to a bulge, or aneurysm.

Small aneurysms generally do not cause problems, but larger ones can cause blood to clot or an artery to rupture or tear, known as dissection. This is a life-threatening event that requires immediate surgery.

There are three types of aneurysms based on their location on the aorta: thoracic, abdominal, and thoracoabdominal.

Aortic aneurysms can be triggered by genetic disorders such as Marfan syndrome (MFS) and related aortic diseases as well as by inflammatory disorders such as giant cell arteritis or atherosclerosis. In all these conditions, cardiovascular risk factors, such as systemic arterial hypertension, may contribute to faster rate of aneurysm progression.

The diagnosis of aortic aneurism is generally made with ultrasound, although a CT scan or an MRI is needed to provide more details about the shape or location of the aneurysm. However, aortic aneurysms can be very difficult to detect through physical examination and may go undetected for years unless specifically tested. In many cases aortic aneurysm has no symptoms, and it is not diagnosed until there is a life-threatening medical emergency.

So, there is an unmet medical need of developing strategies for the diagnosis and treatment of aneurysm. The present invention is focused on solving this problem, and both a method to diagnose aneurysm and a therapeutic strategy are herein provided.

### DESCRIPTION OF THE INVENTION

### Brief description of the invention

As explained above, the present invention refers to an *in vitro* method for the diagnosis of aneurysm which comprises assessing the level of a nucleotide sugar of the hexosamine biosynthetic pathway selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine or of proteins that have been co-translationally or post-translationally modified trough the addition of the above nucleotide sugars. It also refers to an inhibitor of an enzyme involved in the hexosamine biosynthetic pathway selected from the group comprising: GFPT1, GFPT2, GNPNAT1, PGM3 and/or UAP1, and/or of an enzyme downstream the hexosamine pathway selected from: GALE, OGT, OGA, MGAT1, MGAT2, MGAT3, MGAT5B, B3GNT3, GNE, HAS1, HAS3 and/or enzymes involved in the integrated stress response (ISR), for use in the prevention and/or treatment of aneurysm.

To study the pathophysiology of aneurism, the inventors of the present invention have performed experiments with *Fbn1*^{*C1039G*/+} mice, a mouse model of MFS that rapidly develops thoracic aortic aneurysm and dissection (TAAD).

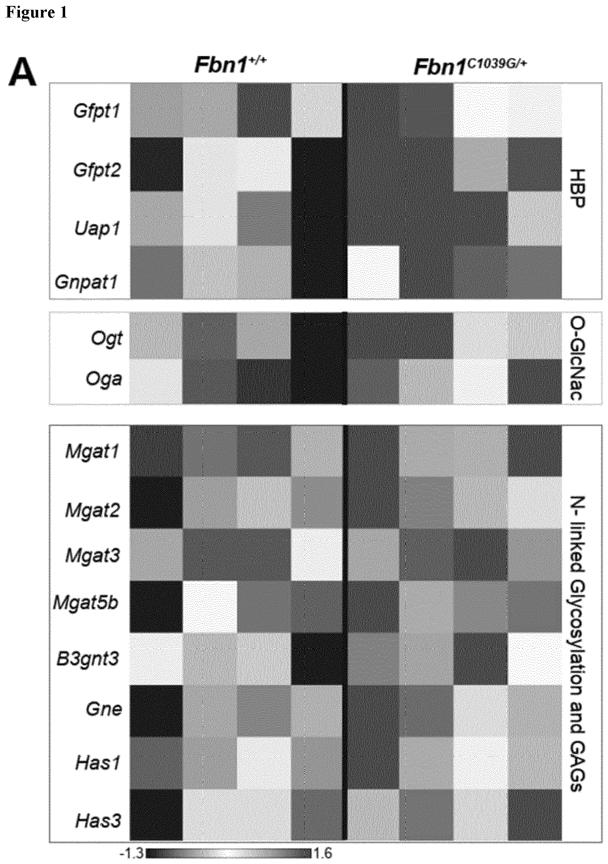

The inventors performed a transcriptomic analysis of the aortas of *Fbn1*^{*C1039G*/+} mice **(****Fig. 1A-**C). This analysis revealed that, compared to aortic tissue from *Fbn1*^{*+*/*+*} mice, aortic tissue from *Fbn1*^{*C1039G*/+} mice has:
- an increased expression of enzymes involved in the hexosamine biosynthetic pathway (HBP). In particular, the overexpressed enzymes are: *Gfptl, Gfpt2, Uap1* and *Gnpat1.*
- an increased expression of enzymes involved in O-linked-N-acetylglucosaminylation (O-GlcNAcylation), which is a type of glycosylation that occurs when the monosaccharide O-GlcNAc is attached to the oxygen atom of serine or threonine residues of nuclear or cytoplasmic proteins. In particular, the enzymes are: *Ogt* and *Oga.*
- an increased expression of enzymes involved in N-linked glycosylation in aortas of *Fbn1*^{*C4039G*/+} mice. N-liked glycosylation occurs when an oligosaccharide is added onto a nitrogen atom of an asparagine residue of a protein. N-glycosylation often occurs co-translationally, in that the glycan is attached to the nascent protein as it is being translated and transported into the endoplasmic reticulum (ER). In particular, the enzymes are: *Mgat1, Mgat2, Mgat3, Mgat5b, B3gnt3, Gne, Has1 and Has3.*

Of note, the above-mentioned pathways/enzymes are closely related, since the HBP pathway results in the synthesis of the sugar uridine diphosphate N-acetylglucosamine (UDP-GlcNAc), and this is an essential common donor substrate for O-GlcNAcylation as well as GlcNAc-branched N-glycosylation.

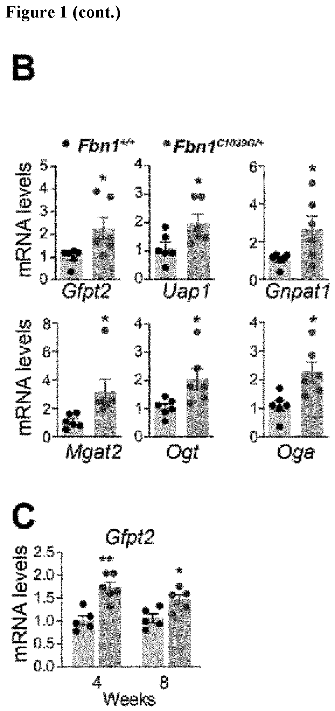

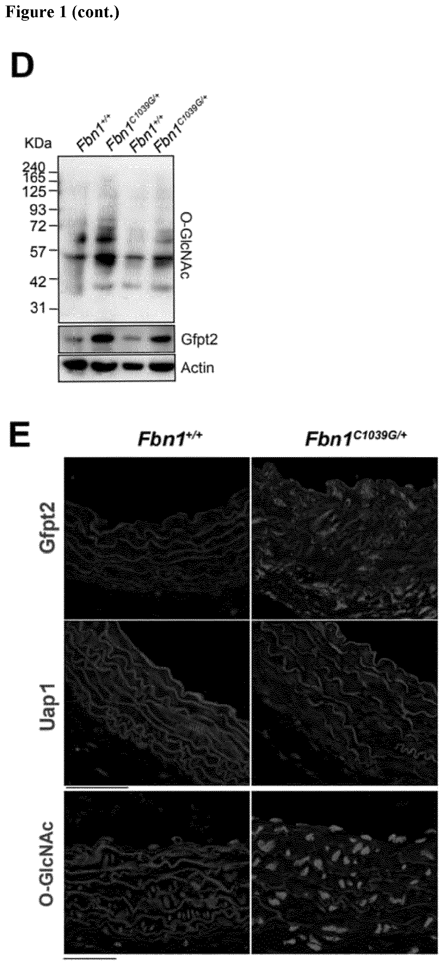

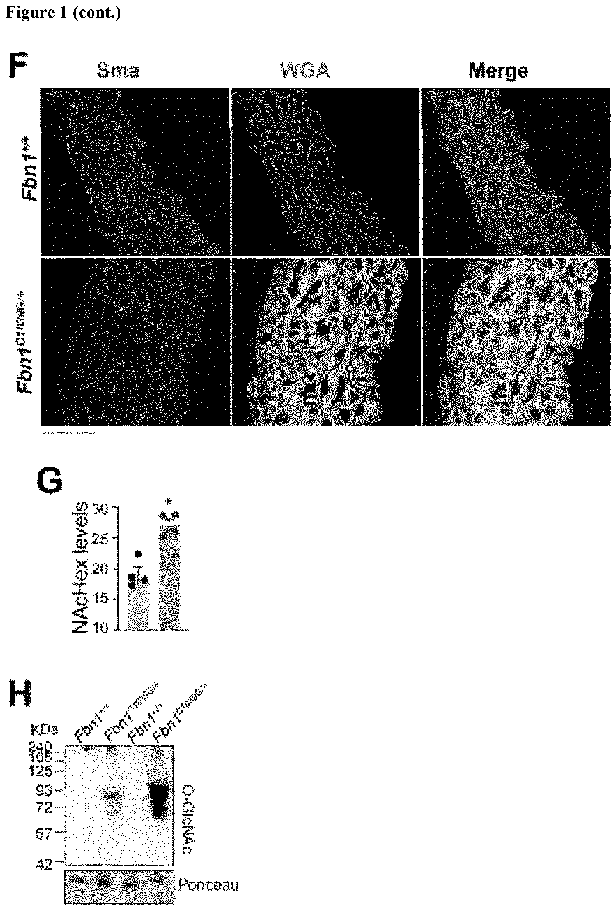

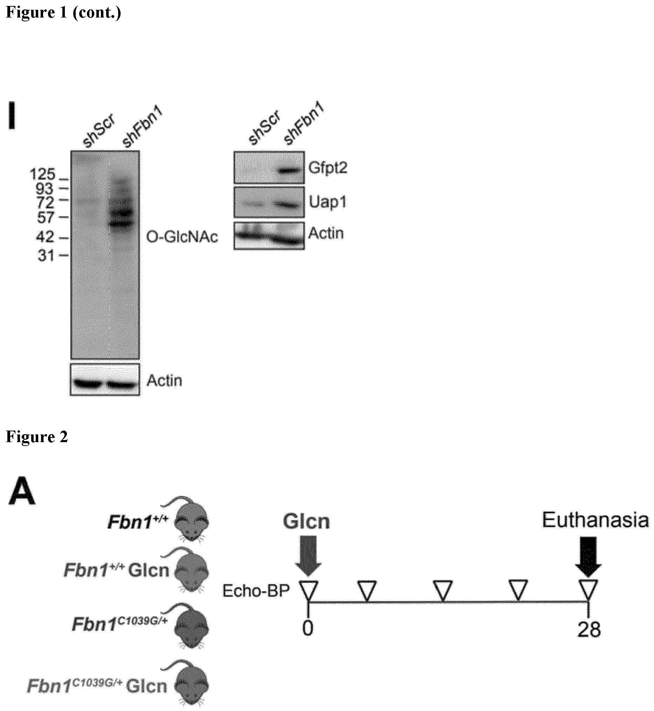

In line with the above results:
- an increased protein expression of GFPT2 and UAP1, as well as an increased content of O-GlcNAc and WGA (a lectin that specifically binds to GlcNAc residues) was detected in aortic tissue from *Fbn1*^{*C1039G*/+} mice compared to controls **(****Fig. 1D-F****).**
- *Fbn1* silencing in primary murine vascular smooth muscle cells (VSMCs) resulted in an increased protein expression of GFPT2 and UAP1, as well as a higher O-GlcNAc content (**Fig. 1I**).
- an increased N-acetylhexosamine and O-GlcNAc content was detected in plasma from *Fbn1*^{*C1039G*/+} mice compared to controls **(****Fig.1** **G, H),** indicating that *Fbn1* deficiency resulted in an increased abundance of downstream HBP metabolites in plasma.

These data suggest that TAAD results in an increased expression of the HBP, and downstream enzymes involved in O-GlcNAcylation and N-liked glycosylation in aortic tissue, and in an increased abundance of proteins that have been co-translationally or post-transcriptionally modified trough the addition of the nucleotide sugars produced by the HBP.

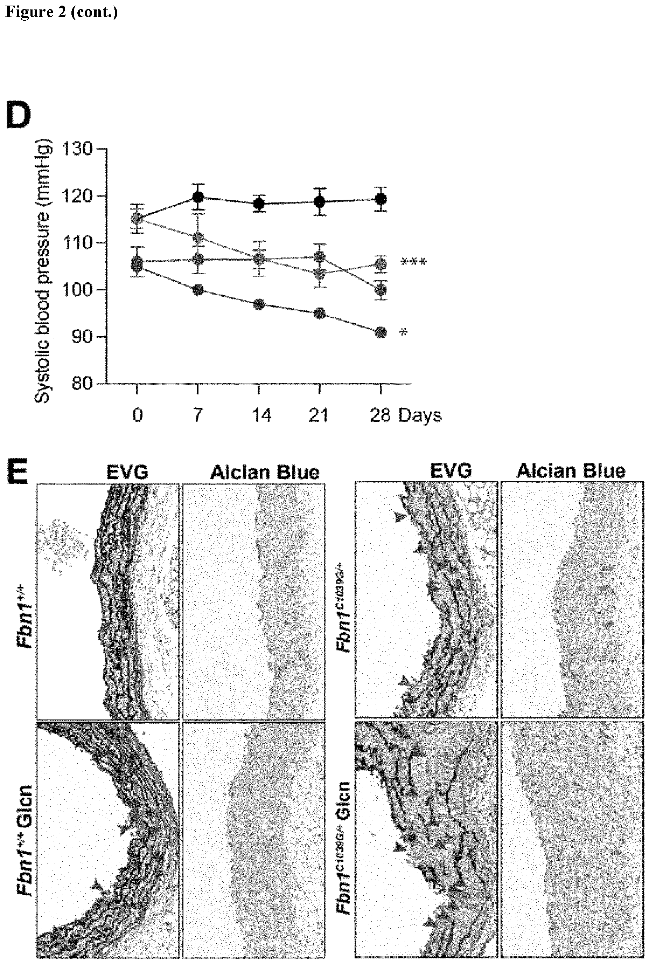

The inventors found that pharmacological induction of HBP increased aortic diameter, decreased blood pressure (BP) **(****Fig. 2A-D****),** and damaged aortic architecture in *Fbn1*^{*C1039G*/+} and control mice **(****Fig. 2E****),** indicating that excessive HBP activity is detrimental for aortic homeostasis.

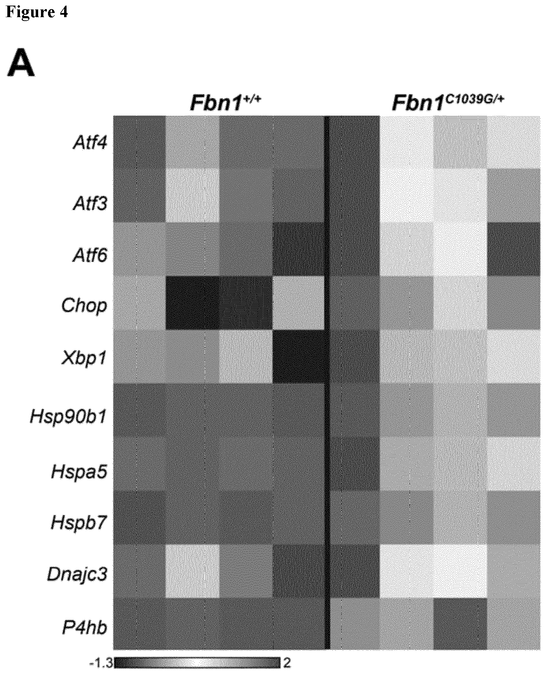

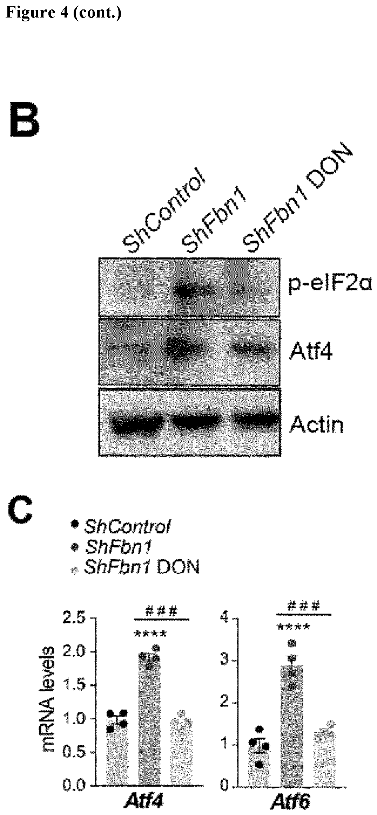

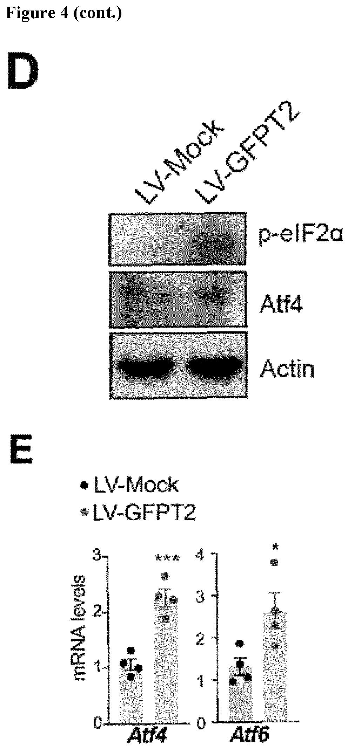

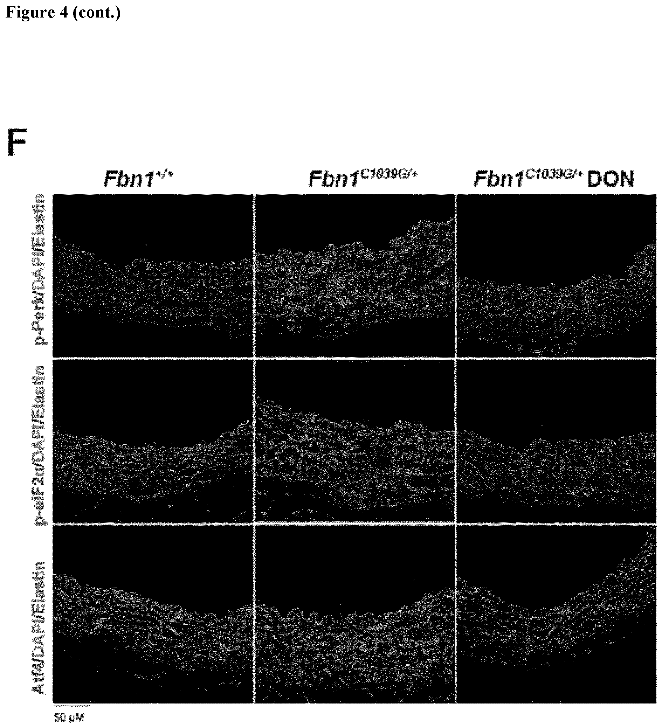

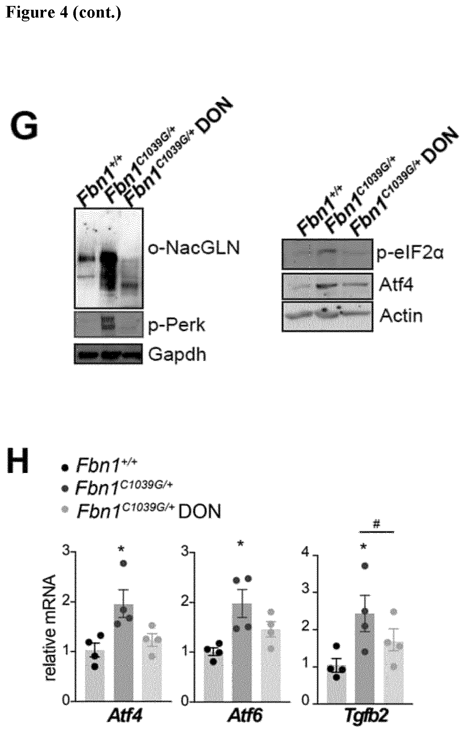

The results provided in the present application suggest that excessive HBP activation leads to the induction of the integrated stress response (ISR) **(Example 2.4).** The inventor found that:
- aortic tissue from *Fbn1*^{*C1039G*/+} mice has an increased expression of genes involved in the ISR compared to their control counterparts **(****Fig. 4A****, F-I),** including an increased protein level of p-PERK, p-eIF2α and ATF4 **(****Fig. 4F****,** **G****).**
- *Fbn1* inhibition induces the IRS through p-eIF2α and ATF4 in VSMCs **(****Fig. 4B, C****).**
- GFPT2 overexpression induces the IRS through p-eIF2α and ATF4 in VSMCs **(****Fig. 4D, E****).**

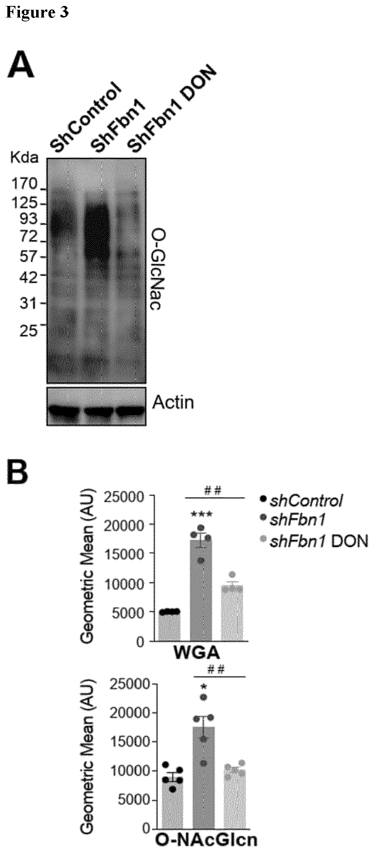

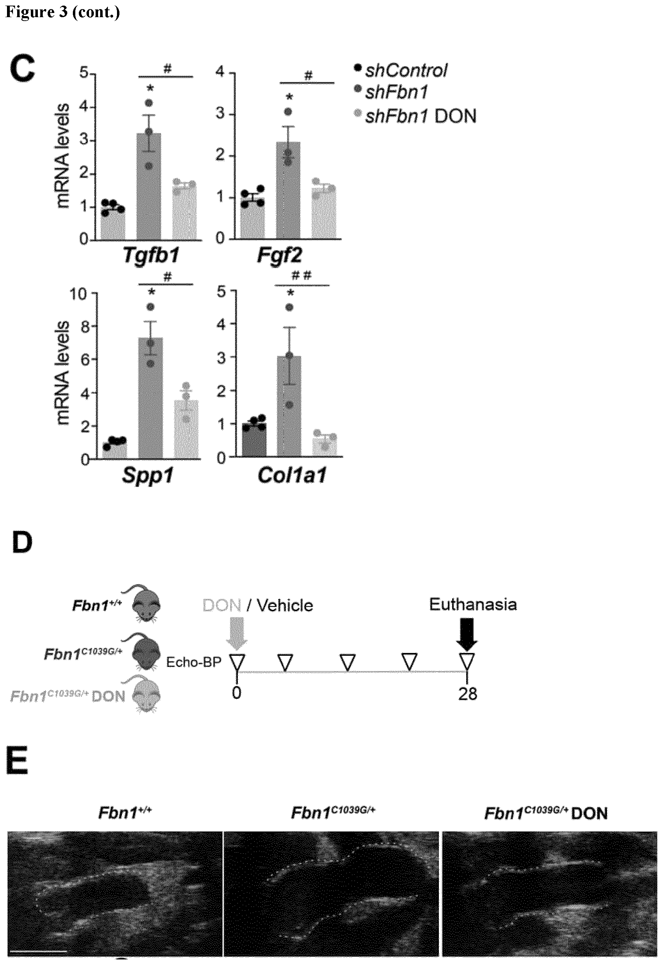

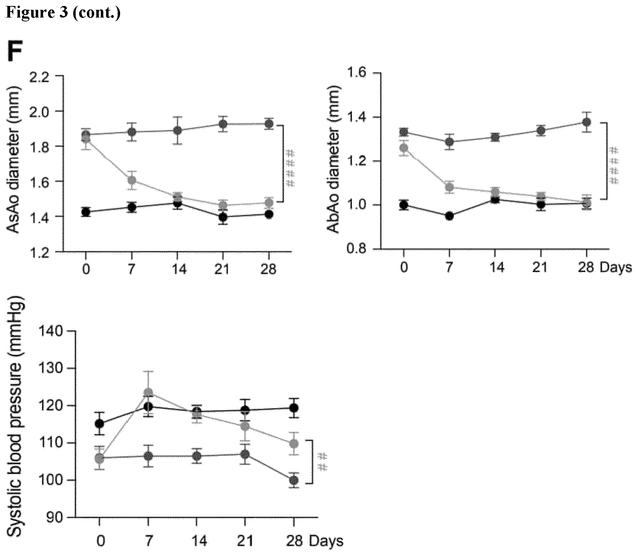

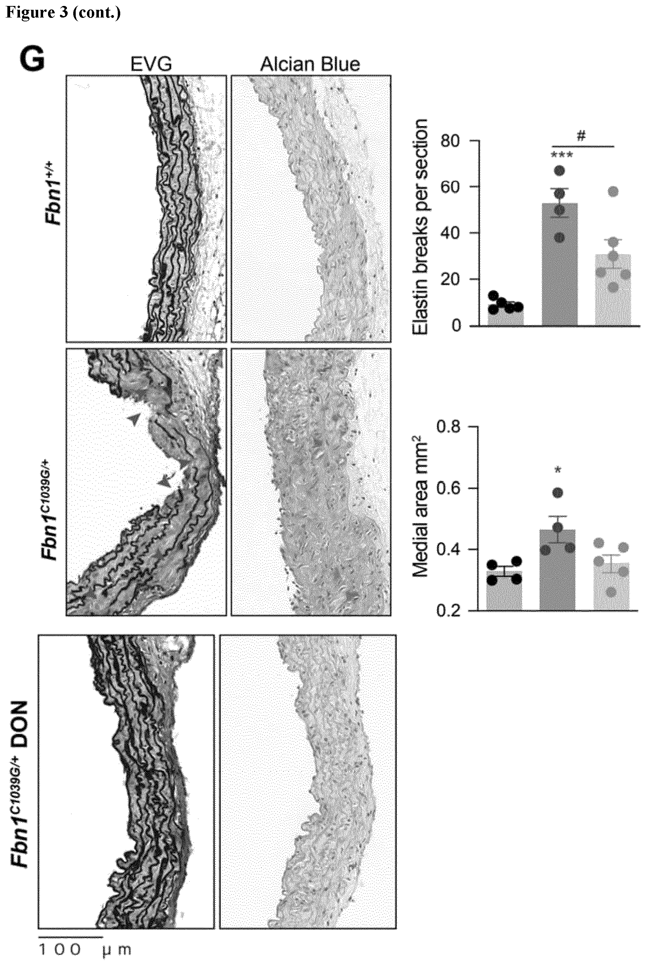

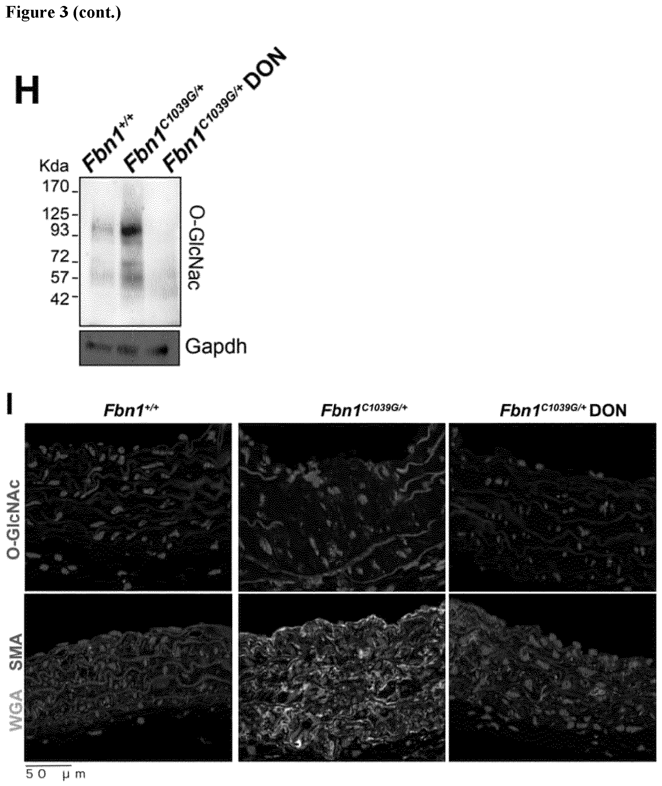

The inventors then investigated the therapeutic potential of HBP inhibition both *in vitro* and *in vivo.* They found that treatment with DON (6-diazo-5-oxo-L-norleucine), an HBS inhibitor that blocks GFPT enzymatic activity:
- reduced O-GlcNAc content in sh*Fbn1*-VSMCs **(****Fig. 3A, B****),** and the expression of classical MFS genes such as *Tgb1, Fgf2, Spp1* and *Col1a1* **(****Fig. 3C****).**
- normalized aortic diameter and blood pressure **(****Fig. 3E****,** **F****),** restored histologic features of aortic degeneration **(****Fig. 3G****),** and reduced O-GlcNAc and WGA content in aortic tissue **(****Fig. 3H, I****)** in *Fbn1*^{*C1039G*/+} mice.
- normalized the expression of genes involved in ISR **(****Fig. 4B****, C, F-J).**

These data indicate that HBP-inhibition improves aortic architecture and homeostasis in a TAAD mouse model.

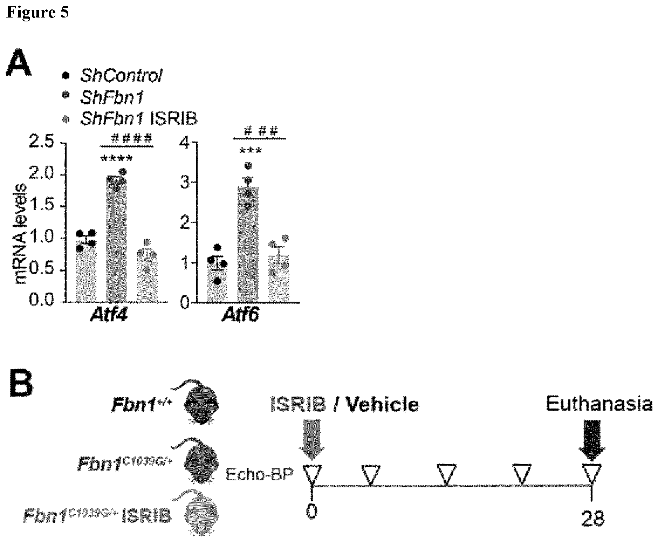

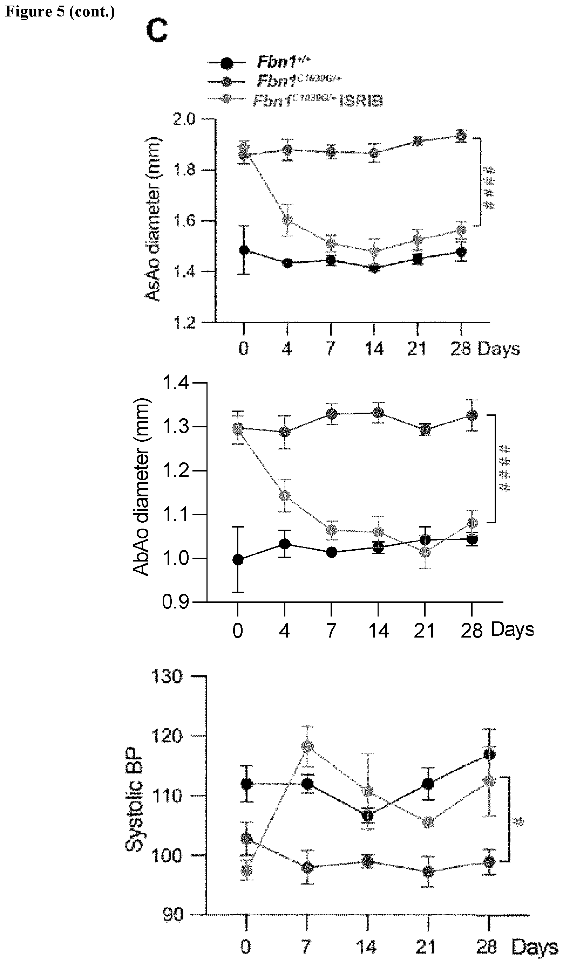

The inventors also investigated the therapeutic potential of inhibiting the ISR both *in vitro* and *in vivo.* They found that treatment with the ISR-inhibitor ISRIB, which is thought to inhibit the ISR through PERK and EIF2a:
- reduced *Atf4, Atf6* mRNA levels in *shFbn1*-VSMCs **(****Fig. 5A****).**
- normalized aortic diameter and systolic blood pressure in *Fbn1*^{*C1039G*/+} mice **(****Fig. 5C****).**

These data indicate that ISR inhibition improves aortic architecture and homeostasis in a TAAD mouse model.

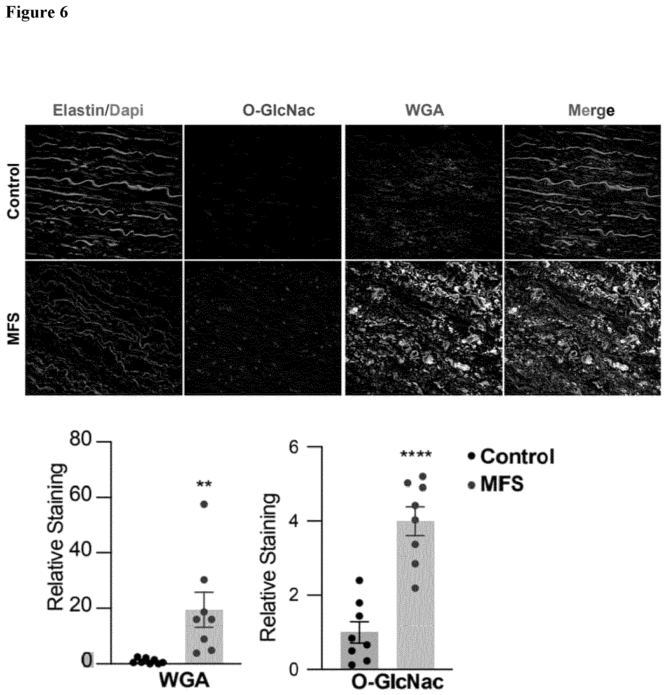

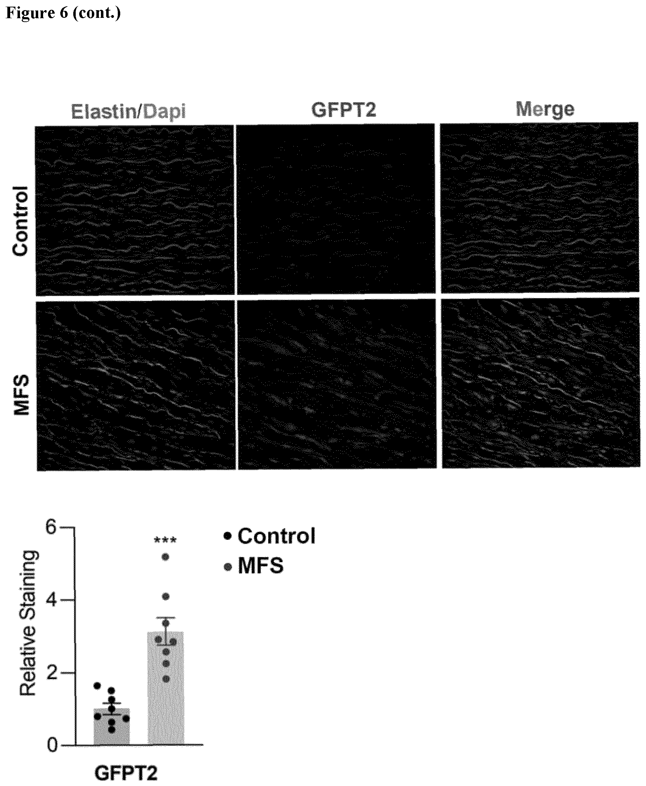

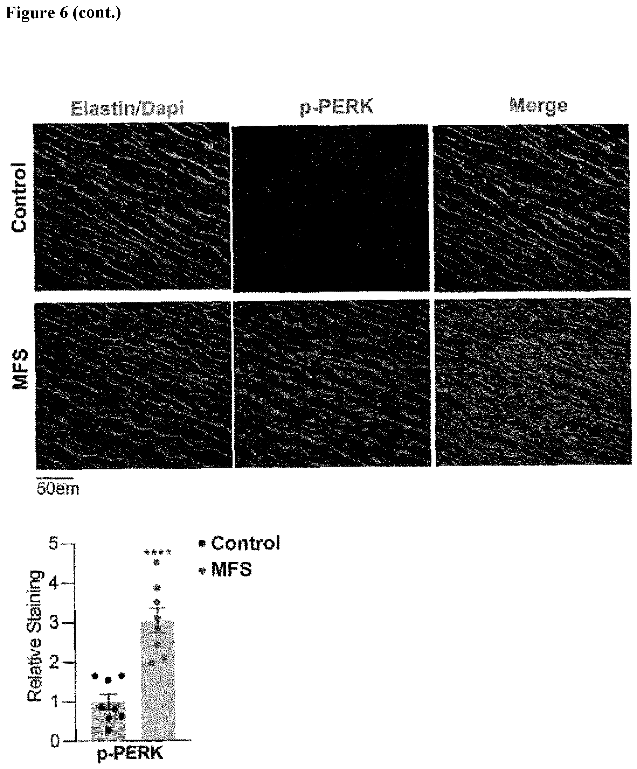

Importantly, the inventors found that aortic sections from MFS-patients also displayed increased levels of GFPT2, O-GlcNAc, WGA and p-PERK **(****Fig. 6****),** suggesting that the findings in the above-mentioned *in vivo* and *in vitro* models are extensive to human TAAD.

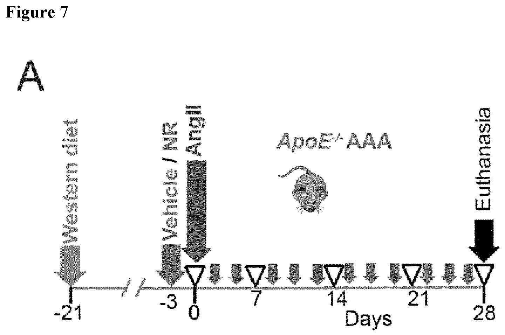

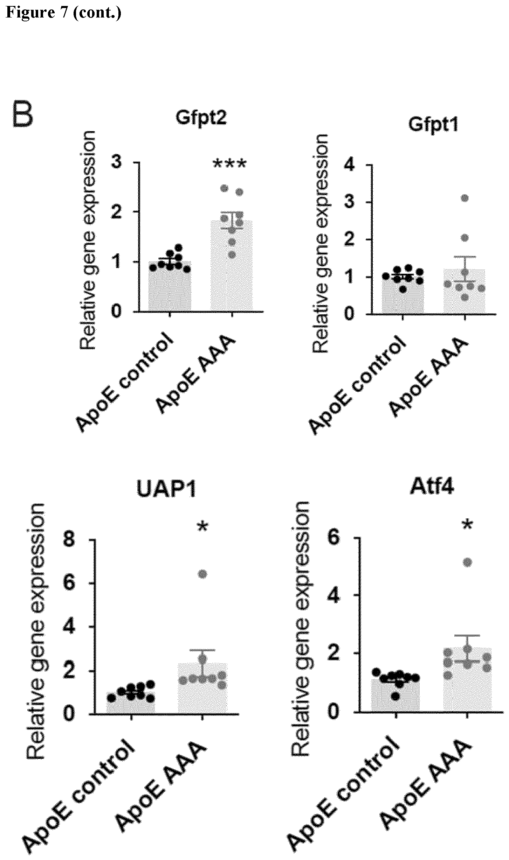

Finally, the inventors assessed the involvement of the HBS and IRS pathways in a mouse model of acute atherosclerotic abdominal aortic aortic aneurysms (AAA) and ruptures. For this, *ApoE-*deficient mice were challenged with angiotensin-II infusion for 28 days at three weeks after the initiation of a high cholesterol and fat diet (*ApoE*^{*-*/*-*} AAA mice, **Fig. 7A****).** In challenged mice, hypertension and hypercholesterolemia promote robust aneurysm development and lethal aortic rupture. *ApoE*^{*-*/*-*} AAA mice displayed increased mRNA expression of HBS markers such as GFPT2 and UAP1, and the IRS master regulator ATF4 compared with *ApoE*^{*-*/*-*} control-mice **(****Fig. 7B****).**

These data indicate that HBS and ISR pathways are also involved in AAA pathology.

So, the first embodiment of the present invention refers to an *in vitro* method for the diagnosis of aneurysm, which comprises:
a) assessing the level of a nucleotide sugar of the hexosamine biosynthetic pathway selected from the list comprising: Glucosamine-6-P, N-acetyl-glucosamine-6P, N-acetyl-glucosamine-1-P or UDP-N-acetyl-glucosamine , Galactosamine-6-P, N-acetyl-galactosamine-6P, N-acetyl-galactosamine-1-P or UDP-N-acetyl-galactosamine, or of proteins that have been co-translationally or post-translationally modified trough the addition of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, in a biological sample obtained from a subject,
b) wherein a higher level, as compared with a pre-established threshold value determined in subjects who are not suffering from aneurysm, is an indication that the subject is suffering from aneurysm.

In a preferred embodiment the biological sample is selected from: aortic tissue, serum, plasma or blood.

In a preferred embodiment the proteins that have been co-translationally or post-translationally modified trough the addition of nucleotide sugars are N-linked glycosylated proteins or O-linked glycosylated proteins, preferably O-linked N-acetylglucosamine acylated (O-GlcNAcylated) proteins or proteins comprising N-acetylhexosamines.

The second embodiment of the invention refers to the *in vitro* use of a nucleotide sugar of the hexosamine biosynthetic pathway selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine or of proteins that have been co-translationally or post-translationally modified trough the addition of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, or of a kit comprising reagents for the detection of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, or of proteins that have been co-translationally or post-translationally modified trough the addition of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, for the diagnosis of aneurysm.

The third embodiment of the invention refers to an *in vitro* method for screening or identifying compounds for use in the prevention and/or treatment of aneurysm, which comprises: a) assessing the enzyme activity of an enzyme involved in the hexosamine biosynthetic pathway selected from: GFPT1, GFPT2, GNPNAT1, PGM3 and/or UAP1, and/or of an enzyme downstream the hexosamine pathway selected from: GALE, OGT, OGA, MGAT1, MGAT2, MGAT3, MGAT5B, B3GNT3, GNE, HAS1, HAS3 and/or an enzyme involved in the integrated stress response (ISR), once the candidate compound has been incubated with the enzyme, and b) wherein if an inhibition of the enzyme is observed, it is indicative that the candidate compound may be effective in the prevention and/or treatment of aneurysm.

The fourth embodiment of the invention refers to an inhibitor of an enzyme (hereinafter referred to as *the inhibitor of the invention*) involved in the hexosamine biosynthetic pathway selected from the group comprising: GFPT1, GFPT2, GNPNAT1, PGM3 and/or UAP1, and/or of an enzyme downstream the hexosamine pathway selected from: GALE, OGT, OGA, MGAT1, MGAT2, MGAT3, MGAT5B, B3GNT3, GNE, HAS1, HAS3 and/or an enzyme involved in the ISR, for use in the prevention and/or treatment of aneurysm.

In a preferred embodiment the enzyme involved in the ISR is selected from the list comprising: PERK and/or EIF2a.

In a preferred embodiment the inhibitor of an enzyme involved in the hexosamine biosynthetic pathway is selected from: azaserine or DON, and/or wherein the inhibitor of the enzyme downstream the hexosamine pathway is selected from: OSMI-1 or ISRIB.

The fifth embodiment of the invention refers to a composition comprising the inhibitor of the invention, optionally, pharmaceutically acceptable excipients or carriers for use in the prevention and/or treatment of aneurysm.

In a preferred embodiment the aneurysm is selected from aortic aneurysm, abdominal aortic aneurysm, preferably abdominal aortic aneurysm associated to atherosclerosis, or thoracic aortic aneurysm, preferably aneurysm associated to Marfan syndrome.

The present invention also refers to:
*In vitro* method for identifying biomarker signatures for the diagnosis of aneurysm, which comprises assessing the level of a nucleotide sugar of the hexosamine biosynthetic pathway selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, or of proteins that have been co-translationally or post-translationally modified trough the addition of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, in a biological sample obtained from a subject, wherein a higher level, as compared with a pre-established threshold value determined in subjects who are not suffering from aneurysm, is an indication that the it is a reliable biomarker.

In a preferred embodiment, the method is focused on detecting biomarkers in a test sample from a human subject at risk of aneurysm.

In a preferred embodiment, the method is complemented by using image techniques like, for example, CT scan or an MRI. Particularly, once an aortic aneurysm is seen or suspected on ultrasound, CT scan or an MRI can be obtained to provide more details about the shape or location of the aneurysm.

In a preferred embodiment, the present invention is a computer-implemented invention, wherein a processing unit (hardware) and a software are configured to: Receive values of any of the above cited biomarkers or signatures, process the values received for finding substantial variations or deviations, and provide an output through a terminal display of the variation or deviation of the concentration level.

### In the context of the present invention the following terms are defined:

- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of'. Thus, the phrase "consisting of" indicates that the listed elements are required or mandatory, and that no other elements may be present.
- The term "pharmaceutically acceptable excipient or carrier" refers to an excipient that may optionally be included with the pharmaceutical composition of the invention and that causes no significant adverse toxicological effects to the patient.
- The expression "proteins that have been co-translationally or post-translationally modified trough the addition of nucleotide sugars" refers to proteins to which sugar residues have added during or after translation. These modifications can be detected using antibodies that recognize the specific nucleotide sugars in the form in that they are bound to the modified proteins. For instance, the O-GlcNAc antibodies recognize GlcNAc only when it has been added to a protein through O-linked glycosylation. Glycosylation is often characterized as a post-translational modification. While this is true with other types of glycosylation, N-glycosylation often occurs co-translationally, in that the glycan is attached to the nascent protein as it is being translated and transported into the ER. This is the reason why the claim mentions "co-translationally or post-translationally".
- The expression "N-linked glycosylated proteins or O-linked glycosylated proteins, preferably O-linked N-acetylglucosamine acylated (O-GlcNAcylated)" refers to proteins to which a nucleotide sugar has been added either through N-linked or O-linked glycosylation. For instance, O-linked N-acetylglucosamine acylated (O-GlcNAcylated) proteins are proteins to which an GlcNAc residue has been added through O-linked glycosylation.

### Description of the figures

**Figure 1****.** Hexosamine biosynthetic pathway is increased in Marfan mouse aortas. **A)** Heatmap showing HBP-related genes from RNAseq performed in aortas from 24weeks-old *Fbn1*^{*+*/*+*} and *Fbn1*^{*C1039G*/+} mice. **B)** Relative mRNA levels analyzed by q-PCR, of HBP-related genes of aortic extracts from 24weeks-old Fbn1^{+/+} and Fbn1^{C1039G}. **C)** Relative *Gfpt2*-mRNA levels from aortic extracts from young *Fbn1*^{*+*/*+*} and *Fbn1*^{*C1039G*/+} mice. **D)** Representative immunoblot of O-glycosylated proteins and Gfpt2 of aortic extracts from 24weeks-old *Fbn1*^{*+*/*+*} and *Fbn1*^{*C1039G*/+} mice. **E)** Representative confocal-immunofluorescence analysis for HBP related proteins and O-glycosylated proteins (Red), Elastin (Green) and Dapi (Blue). **F)** Representative confocal-immunofluorescence analysis for O/N acetyl-glucosilated proteins stained with wheat germ agglutinin (WGA), smooth muscle actin (Red) as marcker for VSMCs. **G)** N-acetyl-galactosamine and N-acetylglucosamine (NAcHex) plasmatic levels from 24weeks-old *Fbn1*^{*+*/*+*} and *Fbn1*^{*C1039G*/+} mice. **H)** Representative immunoblot of O-glycosylated proteins from plasma of *Fbn1*^{*+*/*+*} and *Fbn1*^{*C1039G*/+} mice. **I)** Representative immunoblot of O-glycosylated proteins, Gfpt2 and UAP1 from primary VSMCs silenced for Fbn1 for 5 days.

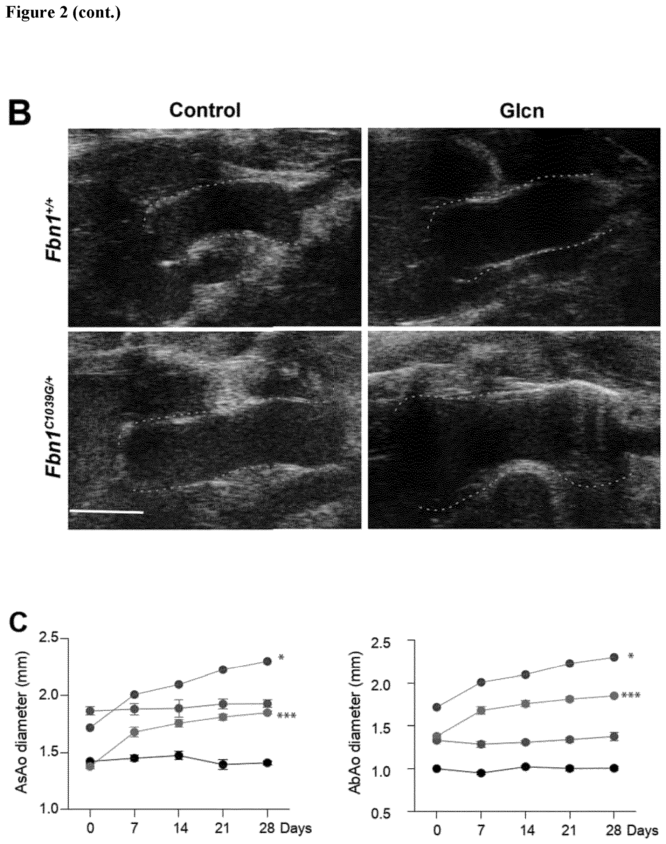

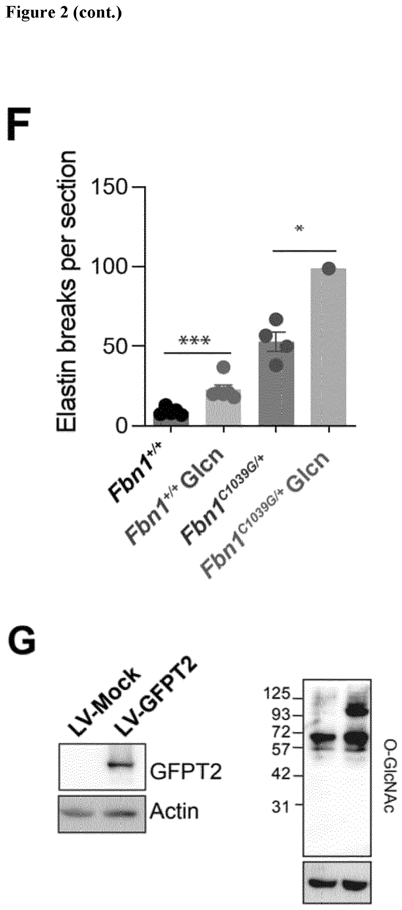

**Figure 2****.** Boosting the hexosamine biosynthetic pathway is deleterious for aortic homeostasis in *Fbn1*^{*+*/*+*} and *Fbn1*^{*C1039G*/+} mice. **A)** Scheme depicting the experimental approach for panels B-E. Glucosamine (Glen) was administrated by drinking water (5%). **B)** Representative ultrasound images of ascending aorta at the end of the experiment. **C)** Aortic diameters along the experiment. **D)** Systolic blood pressure along the experiment. **E)** Representative histological analysis of elastin (EVG, in black), proteoglycan accumulation (Alcian blue, blue) in the cohort of mice from A-D panels. **F)** Quantification of elastin breaks in the cohort of mice from A-D panels. **G)** Lentiviral transduction of GFPT2 overexpressing viruses in primary murine VSMCs. Representative immunoblots of GFPT2 and O-glycosylated proteins.
**Figure 3****.** Pharmaceutical inhibition of the hexosamine biosynthetic pathway improves aortic homeostasis in *Fbn1*^{*C1039G*/+} mice. **A)** Representative immunoblot analysis of VSMCs *Fbn1-*silenced, or sh-control incubated with 6-diazo-5-oxonorleucine (GFPT -inhibitor DON, 10nM) for 48h. **B)** Flow-cytometry analysis of O-glycosylated and O/N-Nacetylglycosilated proteins in VSMCs control or *Fbn1*-silenced treated w/o DON. **C)** Relative mRNA levels of typical Marfan upregulated genes from VSMCs Fbn1-silenced1 incubated w/o DON. **D)** Scheme depicting the experimental approach for panels E-I. Mice age was 20weeks-old, DON was administered by osmotic minipumps (5ng/Kg/day). **E)** Representative ultrasound images of ascending aorta at the end of the experiment. **F)** Aortic and systolic blood pressure along the experiment. **G)** Representative histological analysis and quantification of elastin (EVG, in black), proteoglycan accumulation (Alcian blue, blue) in the cohort of mice from A-D panels. **H)** Representative immunoblot of O-glycosylated proteins from aortic extracts in the same cohort of mice showed in panels D-G. **I)** Representative confocal-immunostaining analysis of O-glycosylated proteins (O-GlcNac) and N/o-acetylglucosylated proteins (WGA); SMA was used as control for VSMCs.

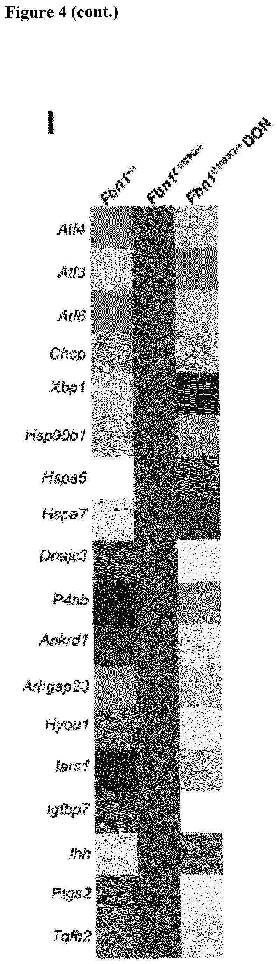

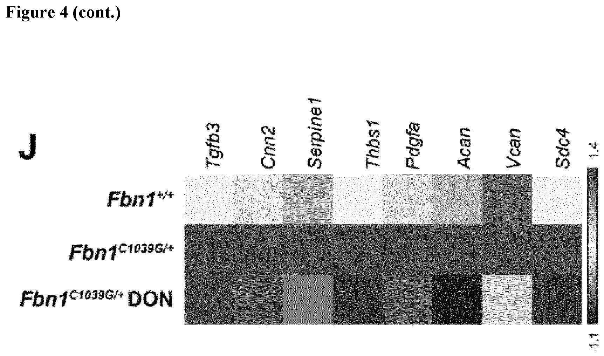

**Figure 4****.** Pharmaceutical inhibition of Hexosamine biosynthetic pathway reduces Integrative Stress Response (IRS) in *Fbn1*^{*C1039G*/+} mice. **A)** Heatmap showing IRS-related genes from RNAseq performed in aortas form the cohort of mice showed in Fig. 1. **B)** Representative immunoblot analysis and **C)** qPCR analysis, IRS-markers from VSMCs *Fbn1*-silenced incubated w/o DON for 48h and **D)** representative immunoblot analysis of IRS-markers and **E)** qPCR analysis, from VSMCs transduced with lentivectors that overexpress GFPT2. **F)** Representative confocal-immunostaining analysis of IRS-markers in the same cohort of mice showed in Fig3. **G)** Representative immunoblot analysis and **H)** qPCR analysis, of IRS-markers from aortic extracts in the same cohort of mice showed in Fig3. **I)** Heatmap of median reads showing IRS-related genes from RNAseq performed in aortas form the cohort of mice showed in Fig.3 (n=4). **J)** Heatmap of median reads showing synthetic VSMCs-phenotype genes from RNAseq performed in aortas form the cohort of mice showed in Fig.3 (n=4).
**Figure 5****.** Pharmaceutical inhibition Integrative Stress Response reduces aortic aneurysm in Fbn1C1039G/+ mice. **A)** qPCR analysis of RS-markers from VSMCs Fbn1-silenced1 incubated w/o DON for 48h and **B)** Scheme depicting experimental approach for panel C. Mice age was 20weeks-old, ISRIB (IRS-Inhibitor) was administrated by osmotic minipumps (10mg/Kg/day). C) Aortic and Systolic blood pressure along the experiment.
**Figure 6****.** Aortic sections from MFS-patients show an increase of HBP and IRS pathways in aortic. Representative confocal-immunostaining analysis of O-glycosylated proteins (O-GlcNac) and N/O-acetylglucosylated proteins (WGA); GFPT2 and p-PERK in aortic media of control and MFS-patients SMA was used as control for VSMCs.
**Figure 7****.** The HBS and IRS are increased in a murine model of atherosclerotic-abdominal aortic aneurysm (AAA). **A)** Scheme of the experiment, *ApoE* null mice were fed with high cholesterol and fat diet (western-diet) 21-days prior AngII infusion. Abdominal aortic diameter after AngII infusion. **B)** Relative mRNA levels analysed by qPCR of HBS and IRS markers in *ApoE*^{*-*/*-*} AAA mice and *ApoE*^{*-*/*-*} controls (without AngII and western diet).

### Detailed description of the invention

The present invention is illustrated by means of the Examples set below without the intention of limiting its scope of protection.

### Example 1. Material and Methods

### Example 1.1. Animal Procedures

Marfan mouse model which harbor a *Fbn1*^{*C1039G*/+} allele (JAX stock #012885). Wild-type littermates were used as controls for Marfan mice. DON dissolved in saline, (Sigma-Aldrich), at 5 ng* kg⁻¹ min⁻¹ and ISRIB (MedChemExpress) at 10mg* kg⁻¹ min⁻¹ were infused using subcutaneous osmotic minipumps (Model 2004, Alzet Corp). NR (Novalix) was administered intraperitoneally at 1000 mg/kg in saline every other day. Mice were housed at the pathogen-free animal facility of the CNIC and CBMSO following the animal care standards of the institution. Animal procedures were approved by the CNIC and CBMSO-UAM Ethics Committee and the Madrid regional authorities and conformed with EU Directive 2010/63EU and Recommendation 2007/526/EC regarding the protection of animals used for experimental and other scientific purposes, enforced in Spanish law under Real Decreto 1201/2005.

The ApoE (Apolipoprotein E)-deficient mice, B6.129P2-Apoetm1Unc/J (JAX stock No. 002052), in pure C57BL6J background were obtained from Charles Rivers. To accelerate atherosclerosis and increase the rate of aortic ruptures, 12-week-old male mice were fed a western diet (high fat and cholesterol diet, SSNIFF-S9167-E011, Ssniff Spezialdiäten, Germany), 3 weeks prior and maintained to Ang-II (angiotensin-II) infusion. Only male mice were studied as females are protected from developing AAA. Ang-II was dissolved in saline (Sigma-Aldrich) and infused at 1 µg/kg·min using subcutaneous osmotic minipumps (Model 2004, Alzet Corp.).

### Example 1.2. Blood pressure measurements and in vivo imaging

Arterial blood pressure (BP) was measured by the mouse-tail cuff method using the automated BP-2000 Blood Pressure Analysis System (Visitech Systems, Apex, NC, USA). Mice were trained for BP measurements every day for five consecutive days. After training period, BP was measured one day before treatment to determine the baseline BP values in each mouse cohort. BP measurements were recorded in mice located in a tail-cuff restrainer over a warmed surface (37 °C). Fifteen consecutive systolic and diastolic BP measurements were made, and the last 10 readings per mouse were recorded and averaged. For *in vivo* ultrasound images, the aortic diameter was monitored in isoflurane-anesthetized mice (2% isoflurane) by highfrequency ultrasound with a VEVO 2100 echography device (VisualSonics, Toronto, Canada) at 30-micron resolution. Maximal internal aortic diameters were measured at diastole using VEVO 2100 software, version 1.5.0.

### Example 1.3. Cell procedures

Isolation and culture of primary mouse VSMCs were previously described. Tissue was digested with a solution of collagenase and elastase (Worthington Biochem) until a single-cell suspension was obtained. All experiments with primary VSMCs were performed during passages 2-7. Lentiviral transduction was performed in VSMCs over 5 h at a multiplicity of infection = 3. The medium was then replaced with fresh DMEM supplemented with 10% FBS and cells were cultured for five more days, treated with NR for five more days and serum-starved for 16h. The HEK-293T (CRL-1573) and Jurkat (Clone E6-1, TIB-152) cell lines, required for high-titer lentivirus production and lentivirus titration respectively, were purchased from ATCC. The experiments were performed during passages 5-10. All cells tested negative for *Mycoplasma.*

### Example 1.4. Lentivirus production and infection

The GFPT2 and GFP coding sequence was obtained from Origene. Lentiviruses expressing shRNA targeting murine *Fbnl,* and control shRNA were purchased from Sigma-Aldrich. Pseudo-typed lentiviruses were produced by transient calcium phosphate transfection of HEK-293T cells and concentrated from culture supernatant by ultracentrifugation (2 hours at 128,000xg; Ultraclear Tubes; SW28 rotor and Optima L-100 XP Ultracentrifuge; Beckman). Viruses were suspended in cold sterile PBS and titrated by transduction of Jurkat cells for 48h. Transduction efficiency (GFP-expressing cells and Puromycin resistant-cells) and cell death (propidium iodide staining) were quantified by flow cytometry.

### Example 1.5. Real-time and quantitative PCR

Aortas were extracted after perfusion with 5 ml saline solution, and the adventitia layer was discarded. Liquid nitrogen frozen tissue was homogenized using a cold-mortar and an automatic bead homogenizer (MagNA Lyzer, Roche). Total RNA was isolated with Trizol (Life Technologies). Total RNA (1 µg) was first digested with DNAse and reverse-transcribed with Maxima First Strand cDNA Synthesis Kit (ThermoFisher). For analysis of mtDNA levels, total DNA from cells and tissues was extracted with the SurePrep kit (Fisher Scientific) or Trizol respectively, according to the manufacture's guidelines. qPCR reactions were performed in triplicate with SYBR master mix (Promega), according to the manufacturer's guidelines. To examine probe specificity, we conducted a post-amplification melting-curve analysis. For each reaction, only one melting-temperature (Tm) peak was produced. The amount of target mRNA in samples was estimated by the 2-CT relative quantification method, using *B2M, YWHAZ* and *PP1A* for normalization. Fold ratios were calculated relative to mRNA expression levels from controls.

### Example 1.6. Library preparation and Illumina sequencing

Aortas were extracted after perfusion of cold saline solution and the adventitia layer was discarded. Frozen tissue was homogenized, and total RNA was isolated with Trizol (Roche). RNA from Libraries were prepared according to the instructions of the Kit "NEBNext Ultra Directional RNA Library Prep kit for Illumina" (New England Biolabs), following the protocol "Poly(A) mRNA Magnetic Isolation Module". The input yield of total RNA to start the protocol was >300 ng quantified by an Agilent 2100 Bioanalyzer using a RNA 6000 nano LabChip kit. The obtained libraries were validated and quantified by an Agilent 2100 Bioanalyzer using a DNA7500 LabChip kit and an equimolecular pool of libraries was titrated by quantitative PCR using the "Kapa-SYBR FAST qPCR kit forLightCycler480" (Kapa BioSystems) and a reference standard for quantification. After processing on the Illumina HiSeq 2500 instrument, FastQ files were generated containing nucleotide data and quality scores for each position. RNA-seq reads were mapped to the *Mus musculus* reference genome, GRCm38.p6, using Hisat2 v2.1.0 software. Reads were then preprocessed with SAMtools v1.7 to transform SAM files into BAM files, and sorted. These files were used as an input for the HTSeq v0.6.1 package, that produces for each sample a file containing the mapped reads per gene (as defined by the *Mus musculus,* GRCm38.96 version, gff file). Once obtained HTSeq read counts, the Bioconductor RNA-Seq workflow was followed to detect the expression differences of genes using the DESeq2 statistical package. Ingenuity Pathway Analysis (IPA) was used to identify cellular biological functions, gene clusters and upstream regulators.

### Example 1.7. Immunoblot

For Western blot (WB) analysis, cells were lysed at 4 °C in RIPA buffer containing protease and phosphatase inhibitors cocktail (Sigma). Proteins were separated by SDS-PAGE and transferred onto 0.45 µm pore size Immobilon PVDF membrane (Millipore). PVDF membranes were blocked with TBS-T (50 mM Tris, 150 mM NaCl, and 0.1% Tween-20) containing 5% (w/v) milk. Membranes were incubated with primary antibodies diluted 1/500 to 1/1000 followed by TBS-T washes and incubation with HRP-conjugated secondary antibodies (GE healthcare). The signal was visualized by enhanced chemiluminescence with Luminata Forte Western HRP Substrate (Millipore) and the ImageQuant LAS 4000 imaging system. The following antibodies were used: anti-O-GlcNac RL2 monoclonal (Abcam), Anti-GFPT2 (Proteintech), anti-UAP 1 (Novus) anti-PERK (Thermo), and anti-pEIF2 and ATF4 (Cell signalling), anti Actin (Abcam) and anti-α-tubulin (Cell Signaling).

### Example 1.8. Aortic histology

After euthanization by CO₂ inhalation, mice were perfused with saline. Aortas were then isolated and fixed in 10% formalin overnight at 4 °C. Paraffin cross-sections (5µm) from fixed organs were stained with Masson-Trichrome, Alcian blue or Verhoeff elastic-van Gieson (EVG), or they were used for immunohistochemistry or immunofluorescence. Elastic fibers were stained with a modified Verhoeff Van Gieson elastin stain kit (Sigma-Aldrich). Elastic lamina breaks, defined as interruptions in the elastic fibers, were counted in the entire medial layer of three consecutive cross-sections per mouse, using 4-16 mice per experiment, and the mean number of breaks was calculated. For immunostaining, the deparaffinized sections were rehydrated, boiled to retrieve antigens (10 mM citrate buffer, Triton-x 0,05%, pH 6) and blocked for 45 min with 10% goat normal serum, 5% horse serum, Triton-x 0,05% and 2% BSA in PBS. Samples were incubated with the following antibodies for immunohistochemistry or immunofluorescence: monoclonal anti-SMA (1/500, C6198, Sigma), WGA-647 (1/2.000 invitrogene) polyclonal anti-GFPT2 (1/300, Proteintech), monoclonal anti-O-GlcNac (1/300, Abcam), polyclonal anti-p-PERK (Thermo) and anti-pEIF2, ATF4 (1/50 Cell Signaling). Specificity was determined by substituting primary antibody with unrelated IgG (Santa Cruz). For immunohistochemistry, endogenous peroxidase and biotin was blocked with 1% H₂O₂-methanol for 10 minutes and with biotin-block kit (Vector Laboratories) respectively. Color was developed in all samples at the same time with DAB (Vector Laboratories), and sections were counterstained with hematoxylin and mounted in DPX (Fluka). For immunofluorescence, secondary antibodies were Alexa-Fluor-546-conjugated goat anti-rabbit and Alexa-Fluor-647-conjugated goat anti-rabbit (BD Pharmigen). For F-actin determination, fixed aortas were embedded in OCT (Tissue-Tek Sakura), 5µm cross-sections were incubated for 30 minutes with 1:1000 Phalloidin-657 (Millipore) after 10 min of incubation with 0.3% Triton X-100 in PBS. Sections were mounted with DAPI in Citifluor AF4 mounting medium (Aname). Images were acquired at 1024 × 1024 pixels, 8 bits, using a Zeiss-LSM800 microscope with 40× oil-immersion objectives.

### Example 1.9. Human samples

The study was approved by the ethics and clinical research committee of Institute de Salud Carlos III and Cantabria University. Aortas for use as controls were obtained anonymously from multiorgan transplant donors after written informed consent was obtained from their families. During preparation of the heart for transplantation, excess ascending aortic tissue was harvested for the study. Samples from patients were obtained during elective or emergency aortic root surgery for aortic aneurysm-dissection. Patient clinical data were retrieved while maintaining anonymity. Tissues were immediately fixed, kept at room temperature for 48 h and embedded in paraffin. DNA and RNA extraction from paraffin sections were performed with All Prep DNA/RNA FFPE Kit (Quiagen).

### Example 1.10. Statistical analysis

Normality of data was checked using Shapiro-Wilk test. F-test was used to assess the equality of variance assumption. Differences between two groups were assessed using unpaired Student's t-test, t-test with Welch's correction for unequal variances, or Mann-Whitney U test where appropriate. Differences in experiments with ≥3 groups were analyzed by one-way, two-way, repeated-measurements two-way analysis of variance (ANOVA) or mixed-effects linear model and Newman's *post hoc* test, as appropriate. For the statistical analysis of RNA-seq data the p-values are corrected using the FDR method, (FDR <0.05). For survival curves, differences were analyzed with the log-rank (Mantel-Cox) test. Multiple linear regression analysis adjusted for age was performed with R software. For all other analysis GraphPad Prism software 9 was used. Statistical significance was indicated as **P* < 0.05, ***P* < 0.01, ****P <* 0.001 and *****P* < 0.0001. Sample size was chosen empirically based on our previous experiences in the calculation of experimental variability; no statistical method was used to predetermine sample size. Before data analysis, outliers were identified and excluded by using the ROUT method (Q value =5%) to identify outliers provided with GraphPad Prism 9.

The numbers of animals used are described in the corresponding figure legends. All experiments were done with at least three biological replicates. Experimental groups were balanced in terms of animal age, sex and weight. Animals were genotyped before experiments, and they were all caged together and treated in the same way. Sex and age are indicated in the figure legends. Appropriate tests were chosen according to the data distribution. Variance was comparable between groups in experiments described throughout the manuscript. For the rest of experiments, no randomization was used to allocate animals to experimental groups, and investigators were not blinded to group allocation during experiments or to outcome assessments.

### Example 2. Results

### Example 2.1. Hexosamine biosynthetic pathway is increased in aortas from a murine model of MFS

The inventors performed a transcriptional analysis by next generation sequencing to unveil an intermediate stage of aortic disease in the aortas of 24-week-old *Fbn1*^{*C1039G*/+} mice, which reproduce the syndromic phenotype, aortic dilatation, aneurysms, and coronary microvascular dysfunction (CMD) seen in MFS patients. By using Ingenuity Pathway Analysis, they identified that the hexosamine biosynthetic pathway is increased in MFS-mice aortas (p-value= 0,047). The HBP is a minor branch of glycolysis, accounting for only 2-5% of total glucose metabolism in homeostasis. The HBP final metabolite is the nucleotide sugar UDP-N-acetylglucosamine (UDP-GlcNAc). This sugar is used to modify intracellular proteins co-translationally or post-translationally (N- or O-glycosylation) or for the synthesis and assembly of glycosaminoglycans (GAGs), proteoglycans (PGs) and glycolipids. The inducible rate-limiting enzyme of HBP, Glutamine-fructose-6-phosphate transaminase 2 (*Gfpt2*), and the downstream enzymes *Uap1* (UDP-N-Acetylglucosamine Pyrophosphorylase 1) and *Gnpat1* (glucosamine-phosphate N-acetyltransferase 1) were significantly increased in MFS-aortas **(****Fig. 1A****).** The pair of enzymes O-GlcNAc transferase (*Ogt*) and O-GlcNAcase (*Oga*)*,* which are involved in O-GlcNAcylation, were upregulated in MFS aortas **(****Fig. 1A****).** These two enzymes mediate the dynamic cycling of O-GlcNAc on a wide array of cytosolic, nuclear, and mitochondrial proteins. Moreover, the inventors found that Golgi-enzymes involved in N-glycosylation such as *Mgat1, Mgat2, Mgat4a, Mgat5b* (*Alpha-1,3 Mannosyl-Glycoprotein 2-Beta-N-Acetylglucosaminyltransferase* 1, 2, 4a, 5b); were upregulated in MFS-aortas **(****Fig. 1A****).** Transcripts of *Gne* (*Glucosamine UDP-N-Acetyl-2-Epimerase*/*N-Acetylmannosamine Kinase),* which initiates the biosynthesis of N-acetylneuraminic acid, a precursor of sialic acid; *B3gnt3 (UDP-GlcNAc:BetaGa1 Beta-1,3-N-Acetylglucosaminyltransferase* 3), which is involved in the biosynthesis of poly-N-acetyllactosamine chains and the biosynthesis of the backbone structure Sialyl Lewis^{X} (CD15s); and *Has1*/*Has3 (hyaluronan synthase 1*/*3),* which synthesizes hyaluronic acid, were increased in MFS-aortas **(****Fig. 1A****).** These data were confirmed by qPCR analysis in aortic extracts from MFS-mice **(****Fig. 1B****).** The inventors performed an analysis of mRNA *Gfpt2* aortic expression at different ages. *Gfpt2* mRNA levels are significantly increased in the very onset of aortopathy at 4weeks-old in *Fbn1*^{*C1039G*/+} mice when they show barely aortic phenotype **(****Fig. 1C****).**

By immunoblot analysis the inventors found that the GFP2 and O-GlcNAc protein levels were increased in aortic samples of MFS-mice **(****Fig. 1D****).**

These data point to an increase of the HBP and downstream enzymes, which might drive an increase of O-GlcNAc, N-glycosylation and GAGs levels in aortas from MFS mice. The inventors assessed HBP-levels by histological analysis of Gfpt2, Uap1, O-GlcNac by RL2 monoclonal antibody **(****Fig. 1E****)** and extracellular N-glycosylation levels by fluorescent-WGA (Wheat germ agglutinin) **(****Fig.1 G)****.** WGA is a lectin that selectively binds to N-acetylglucosamine residues, mainly present in the ECM. Moreover, in blood plasma from MFS-mice, N-acetylhexosamine and O-GlcNAc proteins are increased **(****Fig.1** **G, H).**

To model MFS *in vitro,* the inventors silenced *Fbn1* with lentiviral vectors in primary murine VSMCs. Validating the transcriptomic data from MFS mice, *shFbn1* VSMCs displayed increased mRNA and protein levels of Gfpt2 and Uap1 protein levels **(****Fig. 1I**). Moreover, *shFbn1* VSMCs showed an increased levels of O-GlcNac and WGA staining **(****Fig. 1I**).

These data support that aortas from *Fbn1*^{*C1039G*/+} mice or Fbn1-deficient VSMCs present augmented HBP that rise O-GlcNac and N-glycosylation levels in aortic tissue and N-acetylhexosamine blood plasmatic levels.

### Example 2.2 HBP promotes aortic dilation and medial degeneration in wild type- and MFS- mice

Because ground substance accumulation in CMD is a characteristic of TAAD the inventors hypothesized that an excessive HBP-activity might be involved in the increase of PGs and GAGs that forms the ground-mucoid substance during TAAD development. The inventors treated mice with glucosamine to fuel HBP. Glucosamine enters the cell, it is transformed to glucosamine-6-phosphate and finally, is converted to UDP-GlcNAc. Glucosamine treatment in drinking water in wild-type mice (*Fbn1*^{+/+}) for 28 days significantly increased aortic diameter and decreased systolic blood pressure (BP) after 7 days of treatment **(****Fig. 2A-D****).** Importantly, glucosamine administration in *Fbn1*^{*C1039G*/+} mice increased aortic diameter compared with control *Fbn1*^{*C1039G*/+} mice **(****Fig. 2A-D****).** Glucosamine reproduced histological features of CMD in *Fbn1*^{*+*/*+*} mice and worsen in *Fbn1*^{*C1039G*/+} mice aortic architecture such as elastin fiber fragmentation and amorphous matrix accumulation in Alcian blue staining after 28 days of treatment **(****Fig. 2E-F****).** Lentiviral transduction with GFPT2 increases O-GlcNAc proteins (Fig. 2G)

These results indicate that an excessive HBP activity is involved in accumulation of ground substance in CMD during TAAD development and is detrimental for aortic homeostasis.

### Example 2.3. HBP has critical role in the aortopathy of MFS

To investigate the therapeutic potential of HBP inhibitors in TAAD, the inventors treated sh*Fbn1*-VSMCs with DON (6-diazo-5-oxo-L-norleucine) for 48h. DON inhibits HBP by blocking GFPT enzymatic activity. DON treatment efficiently reduced O-GlcNac in sh*Fbn1-*VSMCs **(****Fig. 3A, B****).** DON incubation decreased mRNA levels of classical MFS genes such as *Tgb1, Fgf2* growth factors, osteopontin (*spp1*) and colagen1 (*Col1a1*) **(****Fig. 3C****).** The inventors next tested the therapeutic potential of DON for 28-days in the development of aneurysm in MFS mice by modulating HBP-activity **(****Fig. 3D****).** Aortic dilation and blood pressure were completely normalized after 7 days of treatment in 24-weeks *Fbn1*^{*C1039G*/+} mice **(****Fig. 3E****,** **F****).** Moreover, DON-treatment restored histologic features of aortic degeneration in MFS, such as medial thickening, elastic fiber fragmentation, GAG and PG deposition by Alcian blue staining after 28 days of DON-treatment **(****Fig. 3G****).** DON treatment for 28 days reduces O-GlcNac levels and WGA staining in aortic tissue **(****Fig. 3H, I****).**

These data indicate that HBP-inhibition declines O-GlcNac and WGA staining, hence, decreasing CMD, improving aortic architecture and homeostasis in an MFS mice model.

### Example 2.4. IRS is induced by HBS in MFS

Analyzing RNA-seq from aortas of 24-week-old MFS-mice, the inventors found that endoplasmic reticulum (ER) stress is activated (p-value 0.032, z-score 1,342). In MFS-mice aortas gene expression of ER-stress profile genes such as *Atf4, Atf3, Atf6, Chop, Xbp1,* chaperones *Hspb1, Hspa5, Hspb7, Dnajc3* and prolyl 4-hydroxylase (*P4hb*) were increased **(****Fig. 4A****).** The activation of the HBP through increase of Gfpt activity was reported to lead to ER-stress trigging the phosphorylation of both PERK (PKR-like endoplasmic reticulum kinase) and eIF2α (eukaryotic initiation factor 2) as well as downstream ATF4 (Activating Transcription Factor 4) translation, identifying the HBP as a modulator of the ISR.

To determine the relation between IRS and HBP in the vasculature, the inventors treated *ShFbn1*-VSMCs with DON for 48h. DON decreased IRS signaling through Atf4, Atf6 protein and mRNA expression **(****Fig. 4B, C****).** To confirm that excessive HBP activity induces IRS in VSMCs the inventors analyzed Atf4 and Atf6 expression in VSMCs transduced with a lentivector that overexpresses GFPT2 (LV-GFPT2); indicating an activation of ISR **(****Fig. 4D, E****).** To assess whether the HBP activity induces ISR in MFS *in vivo,* the inventors performed a histological analysis of p-pERK, p-eIF2α and Atf4 staining in control and DON-treated *Fbn1*^{*C1039G*/+} mice (same cohort of mice showed in **Fig. 3D****) (****Fig. 4F****).** Moreover, the inventors analyzed by immunoblot O-GlcNAc, p-PERK, p-eIF2α and ATF4 protein levels **(****Fig. 4G****)** and mRNA levels of *Atf4* and *Atf6;* and also, Atf4-response gene *Tgfb2* **(****Fig. 4H****)** in aortic extracts from the same cohort of mice showed in **Fig. 3D****.** These data indicate that ISR is activated in MFS-mice and inhibited by DON treatment.

To characterize the effect of DON treatment at the molecular level, the inventors performed RNA-sequencing on aortas from MFS and control mice in the same cohort of mice showed in Fig. 3D. DON decreased the expression levels of genes related to ER-stress and ISR such as *Atf4, Chop, Xbpl, Hsp90b1* and Atf4-dependent genes such as *Ankrd3, Arhfap23, Hyou1, Iars1, Igfp7,* Cyclooxygenase-2 (*Ptgs2*) and *Tgfb2* **(****Fig. 4I**). Notably, ingenuity analysis predicts a decreased of TGFB-pathway (TGFB upstream regulator, -9,913 activation Z-score, p-value 2,5710⁻¹¹) in DON treated mice. DON decreased the expression of conventional MFS-affected genes such as transcripts involved in the TGFβ pathway and those encoding ECM-related proteins such as *Tgfb3, Cnn2, Serpine1, Thbs1, Pdgfa, Acan, Vcan Sdc4* **(****Fig. 4J****).** Thus, reducing HBP fully reverts aortic dilation and the associated aortic wall remodeling such as medial ground substance accumulation herein, CMD; and ISR-signaling in aortas from MFS mice.

### Example 2.5. IRS in aortopathy in MFS

ISR is a decisive cell response pathway that controls translation and bases a protective response and drive a transcriptional program to maintain and to contribute to organismal fitness. However, when the stress cannot be mitigated, the ISR contributes to a maladaptive stress response such as senescence, cellular dysfunction and cell death. First, the inventors evaluated the ISRIB (an ISR-inhibitor) in MFS *in vitro.* ISRIB is a small molecule that reverses the consequences of p-eIF2α and it was tested in Alzheimer, traumatic brain injury and aged dementia mouse models. ISRIB incubation in *ShFbn1-VSMCs* for 48h reduces *Atf4, Atf6* mRNA levels **(****Fig. 5A****).**

Then, the inventors evaluated the possible role of ISR in MFS aortic pathology by ISRIB-treatment (ISR-Inhibitor) in MFS-mice for 28 days **(****Fig 5B****).** ISRIB treatment decreases aortic diameter and blood pressure in *Fbn1*^{*C1039G*/+} mice to normal levels **(****Fig. 5C****).** These data suggest that maladaptive ISR-response mediates aortic pathology in TAAD.

### Example 2.6. HBP-ISR axis in aortas from human MFS patients

Next, the inventors assessed whether the contribution of HBP and ISR occurs in MFS-patients. The inventors found that immunostaining of GFPT2 in the medial layer of aortic sections from patients with MFS was significantly increased compared with the aortic sections from organ transplant donors **(****Fig. 6****).** Histologic analysis of O-GlcNac and WGA levels confirmed a marked increase of HBP in human aortic MFS-samples **(****Fig. 6****).** Of note, immunostainings of pPERK indicate a clear activation of ISR in medial aortic samples from MFS-patients **(****Fig. 6****).** Taken together, these data support the idea that HBP-ISR axis might be important mediators of the aortic pathology in human TAAD and warrant evaluation of HBP-ISR inhibitors fort the treatment of TAAD.

### Example 2.7. HBS and IRS are increased in a murine model of atheroslcerotic-abdominal aortic aneurysm (AAA)

To investigate the contribution of the HBS and IRS in a mouse model of acute atherosclerotic abdominal aortic aortic aneurysms (AAA) and ruptures, ApoE-deficient mice were fed on a western diet for 3 weeks and infused with angiotensin-II for 28 days (*ApoE*^{*-*/*-*} AAA mice, **Fig. 7A****).** In challenged mice, hypertension and hypercholesterolemia promote robust aneurysm development and lethal aortic rupture. mRNA levels of HBS markers such as GFPT2 and UAP1 were increased in *ApoE*^{*-*/*-*} AAA mice compared with *ApoE*^{*-*/*-*} control-mice **(****Fig. 7B****).** Moreover, IRS master regulator, ATF4 were increased in AAA-mice compared with *ApoE*^{*-*/*-*} control-mice **(****Fig. 7B****).** These data indicate that also in AAA pathology HBS and IRS were increased.

## Claims

1. *In vitro* method for the diagnosis of aneurysm, which comprises:
a) assessing the level of a nucleotide sugar of the hexosamine biosynthetic pathway selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, or of proteins that have been co-translationally or post-translationally modified trough the addition of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, in a biological sample obtained from a subject,
b) wherein a higher level, as compared with a pre-established threshold value determined in subjects who are not suffering from aneurysm, is an indication that the subject is suffering from aneurysm.

2. *In vitro* method, according to claim 1, wherein the aneurysm is selected from aortic aneurysm, abdominal aortic aneurysm, preferably abdominal aortic aneurysm associated to atherosclerosis, or thoracic aortic aneurysm, preferably aneurysm associated to Marfan syndrome.

3. *In vitro* method, according to any of the previous claims, wherein the biological sample is selected from: aortic tissue, serum, plasma or blood.

4. *In vitro* method, according to any of the previous claims, wherein the proteins that have been co-translationally or post-translationally modified trough the addition of nucleotide sugars are N-linked glycosylated proteins or O-linked glycosylated proteins, preferably O-linked N-acetylglucosamine acylated (O-GlcNAcylated) proteins or proteins comprising N-acetylhexosamines.

5. *In vitro* use of a nucleotide sugar of the hexosamine biosynthetic pathway selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine or of proteins that have been co-translationally or post-translationally modified trough the addition of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, or of a kit comprising reagents for the detection of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, or of proteins that have been co-translationally or post-translationally modified trough the addition of a nucleotide sugar selected from the list comprising: Glucosamine-6-P, N-acetyl-glucosamine-6P, N-acetyl-glucosamine-1-P or UDP-N-acetyl-glucosamine, Galactosamine-6-P, N-acetyl-galactosamine-6P, N-acetyl-galactosamine-1-P or UDP-N-acetyl-galactosamine, for the diagnosis of aneurysm.

6. *In vitro* use, according to claim 5, wherein the aneurysm is selected from aortic aneurysm, abdominal aortic aneurysm, preferably abdominal aortic aneurysm associated to atherosclerosis, or thoracic aortic aneurysm, preferably aneurysm is associated to Marfan syndrome.

7. *In vitro* method for screening and/or identifying compounds for use in the prevention and/or treatment of aneurysm, which comprises: a) assessing the enzyme activity of an enzyme involved in the hexosamine biosynthetic pathway selected from: GFPT1, GFPT2, GNPNAT1, PGM3 and/or UAP1, and/or of an enzyme downstream the hexosamine pathway selected from: GALE, OGT, OGA, MGAT1, MGAT2, MGAT3, MGAT5B, B3GNT3, GNE, HAS1, HAS3 and/or an enzyme involved in the integrated stress response (ISR), once the candidate compound has been incubated with the enzyme, and b) wherein if an inhibition of the enzyme is observed, it is indicative that the candidate compound may be effective in the prevention and/or treatment of aneurysm.

8. *In vitro* method, according to claim 7, wherein the aneurysm is selected from aortic aneurysm, abdominal aortic aneurysm, preferably abdominal aortic aneurysm associated to atherosclerosis, or thoracic aortic aneurysm, preferably aneurysm associated to Marfan syndrome.

9. *In vitro* method, according to any of the claims 7 or 8, wherein the enzyme involved in the integrated stress response (ISR) is selected from the list comprising: PERK and/or EIF2a.

10. Inhibitor of an enzyme involved in the hexosamine biosynthetic pathway selected from the group comprising: GFPT1, GFPT2, GNPNAT1, PGM3 and/or UAP1, and/or of an enzyme downstream the hexosamine pathway selected from: GALE, OGT, OGA, MGAT1, MGAT2, MGAT3, MGAT5B, B3GNT3, GNE, HAS1, HAS3 and/or an enzyme involved in the integrated stress response (ISR), for use in the prevention and/or treatment of aneurysm.

11. Inhibitor for use, according to claim 10, wherein the aneurysm is selected from aortic aneurysm, abdominal aortic aneurysm, preferably abdominal aortic aneurysm associated to atherosclerosis, or thoracic aortic aneurysm, preferably aneurysm associated to Marfan syndrome.

12. Inhibitor for use, according to any of the claims 10 or 11, wherein the enzyme involved in the integrated stress response (ISR) is selected from the list comprising: PERK and/or EIF2a.

13. Inhibitor for use, according to claims 10 to 12, wherein the inhibitor of an enzyme involved in the hexosamine biosynthetic pathway is selected from: azaserine or 6-diazo-5-oxo-norleucine (DON), and/or wherein the inhibitor of the enzyme downstream the hexosamine pathway is selected from: OSMI-1 or ISRIB.

14. Composition comprising any of the inhibitors of claims 10 to 13 and, optionally, pharmaceutically acceptable excipients or carriers for use in the prevention and/or treatment of aneurysm.

15. Composition for use, according to claim 14, wherein the aneurysm is selected from aortic aneurysm, abdominal aortic aneurysm, preferably abdominal aortic aneurysm associated to atherosclerosis, or thoracic aortic aneurysm, preferably aneurysm associated to Marfan syndrome.
